# Europäisches Patentamt

(19) European Patent Office

Office européen des brevets

(11) Publication number: **0 293 707**
**B1**

(12) **EUROPEAN PATENT SPECIFICATION**

(45) Date of publication of the patent specification:
27.12.90

(21) Application number: 88108209.3

(22) Date of filing: 21.05.88

(51) Int. Cl.⁵: **C07D 303/04**, C07D 301/03, C07D 301/02

(54) Process for preparing propylene oxide.

(30) Priority: 30.05.87 DE 3718266

(43) Date of publication of application:
07.12.88 Bulletin 88/49

(45) Publication of the grant of the patent:
27.12.90 Bulletin 90/52

(84) Designated Contracting States:
BE DE FR GB IT NL

(56) References cited:
EP-A- 0 117 147
US-A- 4 297 287

(73) Proprietor: THE DOW CHEMICAL COMPANY, 2030 Dow Center Abbott Road P.O. Box 1967, Midland Michigan 48640-1967(US)

(72) Inventor: Hollmann, Hans-Günther, Bei den Kiefern 4, D-2166 Dollern(DE)
Inventor: McConchie, Garner Earl, 5730 North Shore Drive, Baton Rouge, LA 70817(US)
Inventor: Mark, Frank E., Am Schaferstieg, D-2160 Stade(DE)

(74) Representative: Sternagel, Hans-Günther, Dr. et al, Patentanwälte Dr. Michael Hann Dr. H.-G. Sternagel Sander Aue 30, D-5060 Bergisch Gladbach 2(DE)

ACTORUM AG

## Description

The present invention relates to a process for preparing propylene oxide from a propylene glycol diester by hydrolyzing the propylene glycol diester at least partially to the corresponding hydroxyester and eliminating carboxylic acid from the hydroxyester.

Such a process is known from U.S. Patents 4,012,423; 4,012,424; 4,399,295 and 4,158,008.

From U.S. Patent 4,012,424 it is known to hydrolyze a mixture containing mainly a propylene glycol diester to maximize the glycol monoester content therein and to subject the obtained mixture to a cracking step in the vapor phase in the presence of a basic material to produce an oxirane compound.

U.S. Patent 4,399,295 discloses the production of propylene oxide from a propylene hydroxyester by deacyloxylation in the presence of a basic material whereby the reaction is carried out in a dilute phase transport reactor into which is fed a stream of heated catalyst co-current with a feed gas stream comprising the hydroxyester.

U.S. Patent 4,158,008 discloses the preparation of propylene oxide by elimination of a carboxylic acid from a propylene glycol monoester in the presence of a base in the liquid phase in a high boiling solvent. The propylene glycol monoester is obtained by hydrolysis of the corresponding diester.

Much patent literature exists relating to the preparation of an alkylene glycol monoester or diester.

According to U.S. Patent 2,115,905 a glycol diester is prepared by reacting an alkylene dichloride such as ethylene dichloride and an alkali or alkaline earth metal salt of a fatty acid at an elevated pressure in the presence of a small amount of water. Unfortunately, the described reaction comprises a two-phase reaction mixture, i.e., one phase comprising the carboxylic acid salt and the other phase comprising the alkylene dichloride, which results in mixing and handling difficulties as well as reduced organic ester selectivity. In addition, further increases in the yield of the glycol diesters are desired.

Another method for preparing a glycol diester is described in U.S. Patent 4,298,758. The method comprises reacting dichloroisopropyl ether with acetic acid and an alkali metal salt thereof. Unfortunately, due to cleavage of the ether, the reaction product is a mixed reaction product comprising a major portion of dipropylene glycol diacetate and a smaller portion of propylene glycol diacetate. In addition, the monoacetate of propylene glycol and 1-chloro-1'-acetoxy-bis(2,2'-oxy-propane) are present in the reaction product.

Alternatively, U.S. Patent 3,461,156 describes a method for producing carboxylic acid esters by reacting the corresponding acid with a halocarbon in the presence of a alkali metal hydroxide and a mutual solvent consisting of dimethylformamide (DMF), dimethyl sulfoxide (DMSO) or mixtures of the two. The solvent may also comprise water, preferably from 10 to 25 percent by volume. The amounts of water in the DMF and DMSO affect the yield of the carboxylic acid ester. Unfortunately, the stated yields and product purification processes are commercially undesirable.

U.S. Patent 3,510,500 relates to the reaction of an organic halocarbon with a carboxylic acid, optionally in the presence of a catalytic amount of a salt made from the acid. The corresponding carboxylic acid ester and hydrogen halide are produced. However, the production of hydrogen halide is undesirable.

The Recueuil des Travaux chimiques des Pays-Bas Vol. 18 (1899), page 224 teaches that trimethylenedibromide can be reacted with potassium acetate in an alcoholic solution to the corresponding diacetate.

There are also a variety of commercially employed methods for synthesizing organic diesters. For example, in the preparation of carboxylic acid esters, it is known that alcohols will react with a carboxylic acid anhydride, a carboxylic acid or their combination, in the presence of an acid catalyst such as sulfuric or hydrochloric acid, to form a carboxylic acid ester. Unfortunately, the acid catalyst, water as well as a variety of by-products in the resulting reaction product often make recovery of the desired reaction product difficult.

Furthermore, U.S. Patents 4,012,424; 4,399,295 and 4,158,008 which disclose the preparation of propylene oxide from a propylene glycol diester or monoester teach by reference to various further patents that a propylene glycol diester or the corresponding hydroxyester is obtained by the carboxylation of propylene with molecular oxygen in a carboxylic acid medium in the presence of a catalyst. However, the carboxylation of propylene with molecular oxygen in the presence of a carboxylic acid medium has many disadvantages. Propylene/oxygen mixtures are dangerous to handle. Therefore, an inert reaction diluent such as gaseous carbon dioxide or nitrogen is usually added which, however, reduces the reaction rate. The reaction rate is further significantly reduced by water which is produced as a by-product in the reaction and of which the concentration is permanently increasing in a continuous process unless it is separated from the reaction products in an additional step. In U.S. Patents 3,479,395; 3,668,239 and 3,262,969 a tellurium or palladium salt alone or in combination with a iodine or bromine source is suggested as a catalyst for the carboxylation of propylene. However, the catalyst is difficult to handle, in particular it causes many recycling problems. Furthermore, the conversion of propylene in this reaction is rather low. Due to these difficulties, the acetoxylation of propylene with molecular oxygen and acetic acid to produce propylene glycol monoacetate or diacetate has never been carried out in technical or large scale production in spite of the great efforts of those skilled in the art.

Accordingly, the process for producing propylene oxide from propylene glycol monoacetate or diacetate has never been a great success either due to the difficulties to produce propylene glycol monoacetate or diacetate.

Accordingly, it is highly desirable to produce propylene oxide from a propylene glycol diester or the corresponding hydroxyester without producing the propylene glycol diester or monoester by the deficient methods known in the art, such as the disadvantageous carboxylation of propylene with molecular oxygen and a carboxylic acid.

Surprisingly, it has been found that a propylene glycol diester can be produced at a high yield from a 1,2-dihalogenopropane by a process described below.

Accordingly, the present invention relates to a process for preparing propylene oxide from a propylene glycol diester by hydrolyzing the propylene glycol diester at least partially to the corresponding hydroxyester and eliminating carboxylic acid from the hydroxyester, which process is characterized in that the propylene glycol diester has been produced by reacting a 1,2-dihalogenopropane with an alkali metal or alkaline earth metal salt of a carboxylic acid within a single phase organic liquid reaction diluent wherein the inorganic salt formed during the reaction is insoluble and which reaction diluent contains less than 3 weight percent water, based on the total weight of the reaction mixture.

By the process of the present invention, not only the disadvantages of the carboxylation of propylene with molecular oxygen and a carboxylic acid can be avoided but also starting materials can be used which have a low commercial value. A preferred starting material for producing a propylene glycol diester according to the process of the present invention is 1,2-dichloropropane. 1,2-dichloropropane is a by-product in the propylene oxide production according to the chlorohydrin process. As evidenced by Ullmann's Enzyklopaedie der Technischen Chemie, 4th Edition, Volume 19, pages 474 to 480, the worldwide capacity of the propylene oxide plants in which the chlorohydrin process was used was 1.8 million tons in 1980. In this process 5 to 11 percent 1,2-dichloropropane are produced as propylene oxide, i.e. between 90,000 and 200,000 tons of 1,2-dichloropropane were produced as a by-product in 1980. This by-product however does not have a high commercial value and is often burnt. The burning of these huge amounts of by-products is highly disadvantageous.

According to the present invention propylene oxide is produced from starting materials such as 1,2-dichloropropane which otherwise would be burnt in industry due to their low commercial use.

The first step in the process of the present invention, i.e. the preparation of a propylene glycol diester by reacting a 1,2-dihalogenopropane with an alkali metal or alkaline earth metal salt of a carboxylic acid, is described in detail in the following paragraphs. The reaction diluent, and accordingly the reaction mixture, contains less than three weight percent water, based on the total weight of the reaction mixture.

In German Patent 41,507, it is taught that ethylene dichloride can be reacted with sodium acetate in acetic acid. In the example of German Patent No. 41,507 the reaction of ethylene dibromide with sodium acetate is described in more detail. Ethylene dichloride or dibromide do not tend to produce undesired by-products like a 1,2-dihalopropane. Furthermore, there is no mention of the criticality to remove water from the reaction mixture in order to avoid elimination reactions since it is not important with ethylene dichloride.

In German Offenlegungsschrift DE-A-32 43 545 it is taught that 1,3-diacetoxy-2-methylenepropane can be produced from 1,3-dichloro-2-methylenepropane. The reaction is carried out with acetic acid in the presence of a base or with an alkali or alkaline earth acetate in polar solvents such as acetic acid. 1,3-dichloro-2-methylenepropane does not produce undesirable elimination by-products. Furthermore, there is no mention of the criticality to remove water from the reaction mixture in order to avoid elimination reactions since it is not important with 1,3-dichloro-2-methylenepropane.

When the propylene glycol diesters are produced from their corresponding halocarbons a side reaction is the elimination of hydrogen halide, e.g. HCl. It is known that elimination reactions of 1,2-dichloropropane yielding the thermodynamically very stable chloropropenes are much more favored than the elimination reaction of ethylene chloride or bromide. Delta Hf°298 of liquid ethylene dibromide is -19.3 kcal/mol whereas Delta Hf°298 of 1,2-dichloropropane - which is preferred starting material of the process of the present invention - is -48.3 kcal/mol. This demonstrates that ethylene dibromide reacts much more readily to its corresponding diacetate than 1,2-dichloropropane. Elimination reactions do not take place in 1,3-dichloro-2-methylenepropane at all. Accordingly it is indeed surprising that a 1,2-dihalogenopropane can be subjected to a similar reaction as ethylene dibromide, ethylene dichloride or 1,3-dichloro-2-methylenepropane. Surprisingly, it has been found that the propylene glycol diester can be prepared at unexpectedly high yield (unexpectedly high balance of selectivity and conversion) by reacting a 1,2-dihalogenopropane with a carboxylic acid salt in the described organic liquid reaction diluent. Moreover, the high yield can be obtained without using a catalyst. The inorganic salt formed by the reaction is insoluble in the organic liquid and can easily be removed by filtration or centrifugation.

Since the reaction proceeds using the method according to the invention without the production of significant amounts of by-products, once the inorganic salt has been removed, the desired carboxylic acid diester is readily recovered at high purity using distillation techniques.

The halogen atoms in 1,2-dihalogenopropane preferably the chlorine or bromine, most preferably chlorine.

Preferred carboxylic acid salts are the alkali metal and alkaline earth metal salts of a carboxylic acid having from 2 to 6 carbon atoms, more preferably from 2 to 3 carbon atoms. In general, the carboxylic acid salt is advantageously an alkali metal salt of a carboxylic acid having from 2 to 3 carbon atoms, with a sodium salt or potassium salt of a carboxylic acid having from 2 to 3 carbon atoms being preferred. Most preferably, the sodium salts of acetic acid or propionic acid are employed as carboxylic acid salts in the practice of the present invention.

The halocarbon and carboxylic acid salt are reacted in an organic liquid. Suitable organic liquid reaction diluents in the practice of the present invention are those organic liquids which are capable of forming a single liquid phase with the halocarbon and the carboxylic acid salt and in which the inorganic salt formed by the reaction of the halocarbon with the carboxylic acid salt is insoluble. As used herein regarding the inorganic salt and the organic liquid, the term "insoluble" means that less than 2 weight percent of the salt will dissolve in the organic liquid, based on the weight of the liquid, preferably less than 1 percent, more preferably less than 0.5 percent.

It is generally preferable to use the carboxylic acid, in acid form, which corresponds to the carboxylic acid salt employed. For example, if sodium acetate is employed in preparing the organic diesters, acetic acid is most preferably employed as the organic liquid reaction diluent. When a carboxylic acid is employed as the organic liquid reaction medium, it does not act as a true diluent in that the acid and the acid salt are capable of being dissociated. Therefore, it is generally preferable to employ a carboxylic acid which corresponds to the carboxylic acid salt as this will not lead to mixed esters with a coincident decrease in selectivity. Furthermore, various glycols, glycol ethers and glycol esters can be employed as the organic liquid reaction medium. In general, using these liquids reduces the conversion of the reaction.

However, it may be advantageous to use propylene glycol as a solvent. From the solvent/diester mixture the corresponding monoester can be prepared and propylene oxide is prepared in a further step.

In general, the carboxylic acid salt and halocarbon are empolyed in amounts such that the reaction mixture contains from 0.1 to 2 mols of the carboxylic acid salt per mol of halocarbon. Preferably, the reaction mixture contains from 0.2 to 1.8 mols, more preferably from 0.3 to 1.6 mols, of the carboxylic acid salt for each mol of the halocarbon. Most preferably, the halocarbon is employed in stoichiometric excess of two to three molar when compared to the carboxylic acid salt.

The organic liquid reaction diluent is employed in amounts sufficient to form a single liquid phase with the carboxylic acid salt and the halocarbon. Alternatively, the carboxylic acid salt can be employed in excess amounts which exceed the solubility of acid salt in the organic liquid. Then, as the reaction proceeds, the undissolved (i.e., excess) amounts of acid salt dissolve due to the reaction of the solubilized carboxylic acid salt with the halocarbon. In general, the acid salt is completely dissolved in the organic liquid and the minimum amounts of the organic liquid employed are thus limited to the saturation point of the carboxylic acid salt in the organic liquid at the temperature of operation. For example, sodium acetate will form about a 55 weight percent solution in acetic acid at 180°C and the concentration of the sodium acetate is selected accordingly. In general, the organic liquid reaction diluent is employed in an amount sufficient to form from a 10 to 85 weight percent solution of the carboxylic acid salt, most preferably from 15 to 75 weight percent solution of the carboxylic acid salt.

The presence of low amounts of water in the reaction diluent, and accordingly in the reaction mixture, has also been found to significantly affect the rate and selectivity of the reaction. Specifically, water in the reaction mixture, although increasing the rate of reaction, will more significantly decrease the selectivity of the reaction. Preferably, the amounts of water in the reaction mixture are minimized, with no measurable amounts of water being most preferred. However, in commercial operation, it is often impractical and/or impossible to eliminate water from the reaction mixture and up to three weight percent water based on the total weight of the reaction mixture can be tolerated while still achieving a desirable selectivity. Preferably, the reaction diluent, and accordingly the reaction mixture, contains less than 0.5, more preferably less than 0.2, weight percent water, based on the total weight of the reaction mixture.

In conducting the reaction to prepare the desired organic diester, the halocarbon, carboxylic acid salt and organic liquid are mixed and subjected to an elevated temperature and pressure sufficient to cause reaction. The temperature and pressure most advantageously employed in conducting the reaction are dependent on a variety of factors including the specific reactants and organic liquid reaction diluent employed and the desired reaction times. In general, temperatures from 160°C to 300°C are advantageously employed to give a reasonable rate of reaction (e.g., 90 percent or more conversion in 12 hours or less) coupled with a high selectivity (e.g., selectivity of more than 80 percent). Higher temperatures have been found to reduce selectivity due to the competing elimination reaction. More preferably, the reaction is conducted at a temperature from 170°C to 280°C and most preferably from 180°C to 260°C.

In general, reaction times will vary from 0.1 to 12 hours, with the shorter reaction times generally being employed using higher reaction temperatures. Preferably, the reaction will be conducted for a period of from 0.5 to 8 hours. Preferably more than 99 percent of the used salt of the carboxylic acid should have been reacted.

In general, the reaction is conducted in a closed reactor under a pressure greater than or equal to the vapor pressure of the contents.

In conducting the reaction, the halocarbon, carboxylic acid salt and organic liquid are advantageously mixed, preferably continuously mixed during reaction. A good mixing of the components if preferred since it has been found that better mixing will reduce the time required for the desired reaction.

Following complete reaction and removal of the precipitated inorganic salt formed by the reaction, the desired organic diester is easily recovered, at desired purity, using conventional techniques. For example, due to the significant temperature differences normally encountered in the boiling points between the halocarbon, organic liquid reaction diluent and the dicarboxylic acid diester product, distillation techniques are very often advantageously employed for such recovery. For example, the dicarboxylic acid diester can be separated at a purity of 99 percent or more using distillation techniques. The reaction diluent and the unreacted halocarbon are removed from the distillation column as an azeotropic mixture and recycled into the reaction. Low boiling by-products are recovered from the distillation column. At the end of the reaction the reaction mixture preferably does not contain unreacted salt of the carboxylic acid to avoid its precipitation during the distillation as result of the removal of the reaction diluent.

In addition to the desired dicarboxylic acid diester, the recovered product will contain only small amounts of by-products which can be formed during reaction of the halocarbon and carboxylic acid salt which include elimination products formed by carboxylic acid elimination or dehydrohalogenation.

The produced propylene glycol diester is then at least partially hydrolyzed to the corresponding hydroxyester compound (also designated as glycol monoester compound) in a known way by adding the amount of water required to hydrolyze the diester to the corresponding hydroxyester. Instead of reacting with water, the propylene glycol diester can be transester ficated with propylene glycol. The hydrolysis is for example described in U.S. Patents 4,012,424 and 4,158,008. The hydrolyzation step can be carried out before or during the elimination of carboxylic acid from the hydroxyester described below. However, it is critical that the production of the diester described above is carried out in the absence of the above mentioned amounts of water and water is only added later.

It is not detrimental if a portion of the propylene glycol diester is hydrolyzed to propylene glycol provided that the molar ratio of the diester to the glycol does not become substantially less than 1. A mixture of the propylene glycol diester an propylene glycol behaves as if the hydrozyester were present.

Alternatively, a mixture of the propylene glycol diester an propylene glycol is useful whereby the propylene glycol may have been used as a reaction diluent for producing the diester according to the first step of the present invention. Transesterification of the diester follows in this case the production of the diester. The amount of water which might still be required for hydrolyzing the diester depends on the diester/glycol ratio.

When the propylene glycol diester is hydrolyzed with water only, preferably from 50 to 200 mol percent, more preferably from 80 to 120 mol percent water are used per mol diester.

The hydrolysis may be either acid- or base-catalyzed. Typical acid catalysts are sulfuric acid, sulfonic acids, phosphoric acid, preferably acid ion exchange resins or the acid of the ester of the hydroxyester reactant. Typical base catalysts are alkali metal and alkaline earth metal carboxylates. Catalyst concentrations of from 0.05 to 0.5 mol equivalents are generally used to achieve the desired reaction rate. Catalyst addition is desirable at temperatures below 180°C. Suitable temperatures for the hydrolysis are from 80°C to 250°C, preferably from 130°C to 220°C. Any unreacted water and the carboxylic acid formed as a by-product are preferably isolated from the reaction mixture. The carboxylic acid can be recycled to the first reaction step as a diluent for the propylene glycol diester production from the halocarbon.

From the propylene glycol monoester which is optionally mixed with the corresponding diester and/or glycol or from the mixture of the propylene glycol diester with the propylene glycol, carboxylic acid is then eliminated in a known way, for example as described in U.S. Patents 4,012,423; 4,012,424; 4,399,295 and 4,158,008.

The elimination of carboxylic acid is generally carried out in the presence of a basic material in the vapor or liquid phase.

The bases may be organic or, preferably, inorganic compounds and should be so strongly basic that their 0.1-molar aqueous solution has a pH of at least 8, e.g. from 8 to 13.

Accordingly, alkali metal or alkaline earth metal carbonates, phosphates, borates, aluminates, silicates, oxides or carboxylates, especially potassium acetate and potassium carbonate, may for example be used. Sodium salts are equally suitable. Lithium compounds are also good catalysts.

In liquid phase, the base may be dissolved. In vapor phase, the basic materials may be supported on a neutral or basic carrier such as alpha-alumina, silicon carbide, silicon silicate, zirconium silicate and aluminum silicate.

The elimination of carboxylic acid in the vapor phase is described below in more detail. Reference is made to U.S. Patents 4,012,423; 4,012,424 and 4,399,295.

The hydroxyester, optionally mixed with the glycol, diester or both, may be fed to the reaction zone undiluted or diluted with a carrier gas. The carrier gas may be a liquid at room temperature, such as benzene, toluene, xylene, pseudocumene or water or a non-condensable carrier gas such as nitrogen, helium or carbon dioxide. When a carrier gas is used, the hydroxyesters are from about 10 to 75 percent by weight, preferably from 25 to 60 percent, of the total feed.

The reaction temperature must be sufficient to maintain the hydroxyester in the vapor phase under reaction conditions. Suitable temperature ranges vary depending on the particular hydroxyester, the presence of the carrier gas and the system pressure. Generally, the deacyloxylation or cracking of the hydroxyester proceeds at a temperature of from 250°C to 600°C, peferably from 250°C to 500°C and most desirably from 350°C to 450°C.

Pressures may be up to 28 bars and vacuum down to 0.007 bar. The partial pressure of the hydroxyester should generally not exceed 7 bars. For ease of operation atmospheric pressure is often preferred. It is preferred that the partial pressure of the hydroxyester be from 0.07 to 1.1 bar, most desirably from 0.1 to 0.6 bar.

The residence time of the hydroxyester in the reactor is preferably 0.001 to 20 sec, more preferably 0.2 to 5 sec, calculated based on the empty tube.

It may be desirable to utilize a dilute phase transport reactor as the reaction zone and a fluidized bed for heating and reactivating the basic catalyst when eliminating the carboxylic acid in vapor phase. Such a process is described in detail in U.S. Patent 4,399,295. The process is carried out continuously in the diluent phase transport reactor by:

a) heating a fluidized bed of catalyst in a heating zone;

b) introducing said heated catalyst and a preheated feed gas stream into the reactor, wherein the hydroxyester component of the feed gas is converted to propylene oxide;

c) separating the reacted gas stream from the catalyst.

d) returning the catalyst to the heating zone for reheating; and

e) further processing the reacted gas stream to separate out the propylene oxide.

The elimination of carboxylic acid in liquid phase is described below in more detail. Reference is made to U.S. Patent 4,158,008. The liquid phase reaction is preferred over the gas phase reaction since the former one requires smaller reaction space.

In the liquid phase reaction generally a homogeneous solution of a monoester of 1,2-propylene glycol or a homogeneous solution of a mixture of a propylene glycol diester with water or with propylene glycol alone or together with said monoester is heated at temperatures of from 160°C to 380°C, preferably from 190°C to 350°C, in an organic solvent which boils above the reaction temperature in the presence of a base or a basic-reacting salt of said base and propylene oxide is isolated from the vapor evolved.

The stated mixture are brought into intimate contact and mixed with the solvent which is heated to the reaction temperature and contains the dissolved base, for a brief period, e.g. for from 0.1 seconds to 5 minutes. The reaction mixture is preferably carried out under atmospheric pressure.

The solvents must be inert under the reaction conditions, should boil above the reaction temperature, and must be sufficiently good solvents for all the reactants. This means that in the preferred method of working at atmospheric pressure, the solvent should have a boiling point of above 160°C. Further, it should not contain any functional groups which might react with a reaction product, e.g. with propylene oxide or acetic acid. Accordingly, suitable high-boiling solvents are fairly high molecular weight aliphatic, cycloaliphatic, araliphatic or aromatic non-polar compounds or, preferably, high-boiling polar compounds, above all oxygen-containing and/or nitrogen-containing compounds in which the oxygen and nitrogen atoms are completely substituted.

Specific examples of suitable solvents are a mixture of isomeric triaryldimethanes, dimethyldiphenyl dioxide, 3-methyl-1-phenylindan, N-cyclohexylpyrrolidone, pentaethylene glycol methyl isopropyl diether, alkyltetrahydrothiophene-1, 1-dioxide (where alkyl is, for example, methyl) and, preferably, tetrahydrothiophene-1, 1-dioxide.

The propylene oxide can be recovered from the reaction product gases by cooling them and thereafter distilling the condensate. According to a preferred embodiment of the process of the present invention the carboxylic acid produced as a by-product is recycled to the first reaction step as a reaction diluent for the propylene glycol diester production starting from the corresponding halocarbon. Unconverted diesters, hydroxymonoesters or glycols are preferably recycled to the reactor for elimination of carboxylic acid.

The following examples are presented to illustrate the invention and should not be construed to limit its scope. In the examples, all parts and percentages are by weight, unless otherwise indicated.

### Example 1

#### A. Preparation of a propylene glycol diester

Into a suitably sized, lined pressure contained equipped with an agitator and heating and cooling means are added 180 parts of a water-free acetic acid (i.e., the acetic acid contains less than 1 percent water), 82 parts of sodium acetate and 180 parts of 1,2-propylene dichloride. The mixture is then heated to 200°C and stirred continuously while maintaining this temperature for a period of 6 hours. At this time, the selectivity of the reaction based on the amount of reacted propylene dichloride which forms propylene glycol diacetate (PGDA) is greater than 80 percent calculated from the amount of sodium acetate used. Based on the amounts of sodium chloride found in the reaction product, the conversion is above 90 percent of the sodium acetate added.

Following the reaction, the reaction mixture is cooled to room temperature and filtered to remove the sodium chloride formed by the reaction. The salt-free filtrate contains acetic acid, propylene dichloride, propylene glycol diacetate, monochloropropenes and trace amounts of other materials. The resulting reaction mixture is distilled and a propylene glycol diacetate containing reaction product is separated. This product has greater than 99 percent purity and a boiling temperature of 190°C.

When the foregoing reaction is duplicated, except that the reaction mixture contains 1 percent water based on the total weight of reaction mixture, the selectivity of the reaction to propylene glycol diacetate remains above 70 percent. As the amounts of water in the reaction mixture are increased, the selectivity is found to significantly decrease. Specifically, when the reaction mixture contains 5 percent of water, the selectivity to propylene glycol diacetate is reduced to 50 percent. When the reaction mixture contains 10 percent water, the selectivity has been reduced further to 40 percent whereas at 33 percent water, the selectivity of propylene dichloride to propylene glycol diacetate is only 27 percent based on propylene dichloride.

## Example 2

In a pressure container identical to that used in Example 1 are added 180 parts of sodium acetate and 180 parts of 1,2-propylene dichloride. The mixture is then heated to 220°C and stirred continuously while maintaining this temperature for a period of 4 hours. At this time the selectivity of the reaction based on the amount of reacted propylene dichloride which forms propylene glycol diacetate (PGTA), is greater than 85 percent, calculated from the amount of sodium acetate used. Based on the amount of sodium chloride separated from the reaction product the conversion is above 99 percent of the sodium acetate added.

Following the reaction, the reaction mixture is cooled to room temperature and treated as in Example 1.

The azeotropic mixture of unreacted 1,2-propylene dichloride and acetic acid is recovered from the distillation column and recycled as starting material for further reactions.

## Example 3

Into a pressure container identical to that used in Example 1 are added 180 parts of water-free acetic acid, 82 parts of sodium acetate and 300 parts of 1,2-propylene dichloride. The mixture is then heated to 200°C and continuously stirred while maintaining the temperature for a period of 6 hours. The conversion rate of sodium acetate is above 99 percent determined from the amount of sodium chloride separated from the reaction mixture subsequent to cooling to room temperature. The reaction mixture is then distilled as described in Example 2.

## Example 4

To a reaction vessel identical to that employed in Example 1 are added 54 parts of sodium propionate, 297 parts of propionic acid and 63 parts of 1,2-propylene dichloride. This reaction mixture contains less than 0.5 percent water. It is heated at 200°C, with continuous agitation, for a period of 6 hours. At that time, the conversion of sodium propionate is greater than 80 percent. Using conventional distillation techniques, a product containing propylene glycol dipropionate, at a purity of greater than 99 percent, is recovered. The total yield of propylene glycol dipropionate based on the amount of sodium propionate originally added to the reaction vessel is greater than 65 percent.

## Example 5

180 kg of sodium acetate are added to a mixture of 272 kg of a water-free acetic acid and 235 kg of 1,2-dichloropropane. The reaction is carried out at 220°C, at a pressure of about 13 bar and at a residence time of 150 min. 126 kg of sodium chloride are obtained, which are filtered off, and 147 kg of propylene glycol diacetate.

## Example 6

140 kg of sodium acetate is added to a mixture of 97 kg of a water-free acetic acid and 379 kg of 1,2-dichloropropane. The reaction is carried out at 220°C, at a pressure of about 15 bar and at a residence time of 180 min. 123 kg of propylene glycol diacetate (PGDA) is obtained. The selectivity of the reaction, based on the amount of reacted propylene dichloride which forms PGDA, is bout 88 percent, calculated from the amount of sodium acetate used.

Examples 5 and 6 illustrate that propylene glycol diacetate is obtained from propylene dichloride at a pilot scale at high selectivity.

## B. Preparation of propylene oxide from propylene glycol diacetate

### B1) Vapor phase reaction according to the teaching of U.S. Patent 4,012,424

One hundred grams of propylene glycol diacetate is mixed with 200 grams of water and heated to 185°C. The system pressure rises to 6.4 bar. After five hours the system is cooled and the acetic acid and water distilled overhead. The product mixture is 55 weight percent propylene glycol monoacetate, 33 percent propylene glycol diacetate and 12 percent propylene glycol. This mixture is then fed at a rate of 1.3 grams per minute to a reactor containing a catalyst composed of 40 grams of 11.8 weight percent $K_2Si_2O_5$ on 8-12 mesh alundum operating at 400°C and 333 mbar pressure. 32 percent of the propylene glycol monoacetate react to yield propylene oxide, propionaldehyde and acetone in molar selectivities of 85 percent, 10 percent and 5 percent, respectively.

### B2) Liquid phase reaction according to the teaching of U.S. Patent 4,158,008

One hundred parts of tetrahydrothiophene-1,1-dioxide and 5.0 parts of potassium carbonate are introduced into a stirred vessel provided with a blade stirrer, internal thermometer and dip leg. 0.5 Parts by volume/min. of a propanediol diacetate/propanediol mixture consisting of 67.8 weight percent 1,2-propanediol diacetate and 32.2 weight percent 1,2-propanediol are pumped through the dip leg into the solution at 250°C to 260°C, whilst stirring. The reaction mixture evolved is condensed out in a cold trap (in order to determine the yield) and is examined by gas chromatography. In a 2 hour experiment, 60.0 parts of mixture are employed.

The conversion of the mixture is 72.3 mol percent, based on the amount of propanediol diacetate and 83.7 mol percent, based on the amount of propanediol.

## Claims

1. A process for preparing propylene oxide from a propylene glycol diester by hydrolyzing the propylene glycol diester at least partially to the corresponding hydroxyester and eliminating carboxylic acid from the hydroxyester, characterized in that the propylene glycol diester has been produced by reacting a 1,2-dihalogenopropane, with an alkali metal or alkaline earth metal salt of a carboxylic acid within a single phase organic liquid reaction diluent wherein the inorganic salt formed during the reaction is insoluble and which reaction diluent contains less than 3 weight percent water, based on the total weight of the reaction mixture.

2. The process of Claim 1, characterized in that 1,2-dichloropropane is used for preparing the intermediate propylene glycol diester.

3. The process of Claim 1 or 2, characterized in that the organic liquid reaction diluent is a carboxylic acid and the carboxylic acid salt and carboxylic acid contain from 2 to 6 carbon atoms.

4. The process of Claim 3, characterized in that the carboxylic acid contains the same number of carbon atoms as the carboxylic acid salt.

5. The process of Claim 4, characterized in that the carboxylic acid salt is the sodium or potassium salt of acetic or propionic acid and the organic liquid reaction diluent is acetic acid, propionic acid or 1,2-propylene glycol.

6. The process of any of Claims 1 to 5, characterized in that the organic liquid reaction diluent contains less than 0.5 weight percent water, based on the total weight of the reaction mixture.

7. The process of any of Claims 1 to 6, characterized in that the reaction is conducted at a temperature of from 180°C to 260°C.

8. The process of any of Claims 1 to 7, characterized in that the hydrolyzation of the propylene glycol diester to the corresponding hydroxyester is carried out by reacting the diester with water or with the corresponding propylene glycol and the hydrolyzation takes place before or during the elimination of carboxylic acid from the hydroxyester.

9. The process of any Claims 1 to 8, characterized in that the elimination of the carboxylic acid from the propylene glycol monoester is carried out at a temperature of from 160°C to 380°C in the presence of a base in an organic solvent which boils above the reaction temperature at the prevailing pressure.

10. The process of any of Claims 1 to 9, characterized in that the eliminated carboxylic acid is recycled to the first reaction step as a rection diluent for the production of propylene oxide from a 1,2-dihalogenopropane.

**Patentansprüche**

1. Verfahren zum Herstellen von Propylenoxid aus einem Propylenglycoldiester durch zumindest teilweises Hydrolisieren des Propylenglycoldiesters zum entsprechenden Hydroxyester und Entfernen der Carbonsäure aus dem Hydroxyester, dadurch gekennzeichnet, daß der Propylenglycoldiester hergestellt wurde durch Umsetzen eines 1,2-Dihalogenpropans mit einem Alkalisalz oder Erdalkalisalz einer Carbonsäure in einem einphasigen flüssigen organischen Reaktionsverdünnungsmittel, wobei das während der Reaktion gebildete anorganische Salz im Reaktionsverdünnungsmittel unlöslich ist und das Reaktionsverdünnungsmittel weniger als 3 Gew.-% Wasser, bezogen auf Gesamtgewicht der Reaktionsmischung, enthält.

2. Verfahren nach Anspruch 1, dadurch gekennzeichnet, daß 1,2-Dichlorpropan für die Herstellung des Zwischenproduktes Propylenglycoldiester verwendet wird.

3. Verfahren nach Anspruch 1 oder 2, dadurch gekennzeichnet, daß das flüssige organische Reaktionsverdünnungsmittel eine Carbonsäure ist und das carbonsaure Salz und die Carbonsäure 2 bis 6 Kohlenstoffatome enthalten.

4. Verfahren nach Anspruch 3, dadurch gekennzeichnet, daß die Carbonsäure die gleiche Zahl Kohlenstoffatome enthält wie das carbonsaure Salz.

5. Verfahren nach Anspruch 4, dadurch gekennzeichnet, daß das carbonsaure Salz das Natrium- oder Kaliumsalz von Essigsäure oder Propionsäure ist und das flüssige organische Reaktionsverdünnungsmittel Essigsäure, Propionsäure oder 1,2-Propylenglycol ist.

6. Verfahren nach jedem der Ansprüche 1 bis 5, dadurch gekennzeichnet, daß das flüssige organische Reaktionsverdünnungsmittel weniger als 0,5 Gew.-% Wasser, bezogen auf Gesamtgewicht der Reaktionsmischung, enthält.

7. Verfahren nach jedem der Ansprüche 1 bis 6, dadurch gekennzeichnet, daß die Reaktion bei einer Temperatur von 180°C bis 260°C ausgeführt wird.

8. Verfahren nach jedem der Ansprüche 1 bis 7, dadurch gekennzeichnet, daß die Hydrolyse des Propylenglycoldiesters zum entsprechenden Hydroxyester ausgeführt wird durch Umsetzen des Diesters mit Wasser oder mit dem entsprechenden Propylenglycol und die Hydrolyse vor oder während des Entfernens der Carbonsäure vom Hydroxyester abläuft.

9. Verfahren nach jedem der Ansprüche 1 bis 8, dadurch gekennzeichnet, daß das Entfernen der Carbonsäure aus dem Propylenglycolmonoester bei einer Temperatur von 160°C bis 380°C in Gegenwart einer Base in einem organischen Lösungsmittel ausgeführt wird, das oberhalb der Reaktionstemperatur bei den gegebenen Druckbedingungen siedet.

10. Verfahren nach jedem der Ansprüche 1 bis 9, dadurch gekennzeichnet, daß die abgetrennte Carbonsäure in den ersten Reaktionsschritt zurückgeführt wird als Reaktionsverdünnungsmittel für die Herstellung von Propylenoxid aus einem 1,2-Dihalogenpropan.

**Revendications**

1. Procédé de préparation d'oxyde de propylène à partir d'un diester de propylène glycol par hydrolyse du diester de propylène glycol au moins partiellement en l'hydroxy-ester correspondant et élimination de 1, acide carboxylique de l'hydroxy-ester, caractérisé en ce que le diester de propylène glycol a été produit par réaction d'un 1,2-dihalogénopropane avec un sel de métal alcalin ou de métal alcalino-terreux d'un acide carboxylique, au sein d'un diluant liquide organique pour la réaction, constituant une seule phase, dans lequel le sel inorganique formé durant la réaction est insoluble et qui contient moins de 3% en poids d'eau, par rapport au poids total du mélange réactionnel.

2. Procédé selon la revendication 1, caractérisé en ce que l'on utilise le 1,2-dichloropropane pour préparer le diester de propylène glycol intermédiaire.

3. Procédé selon la revendication 1 ou 2, caractérisé en ce que le diluant organique liquide pour la réaction est un acide carboxylique et que le sel d'acide carboxylique et l'acide carboxylique contiennent de 2 à 6 atomes de carbone.

4. Procédé selon la revendication 3, caractérisé en ce que l'acide carboxylique contient le même nombre d'atomes de carbone que le sel d'acide carboxylique.

5. Procédé selon la revendication 4, caractérisé en ce que le sel d'acide carboxylique est le sel de sodium ou de potassium de l'acide acétique ou de 1, acide propionique et que le diluant organique liquide pour la réaction est l'acide acétique, l'acide propionique ou le 1,2-propylène glycol.

6. Procédé selon l'une quelconque des revendications 1 à 5, caractérisé en ce que le diluant organique liquide pour la réaction contient moins de 0,5% en poids d'eau par rapport au poids total du mélange réactionnel.

7. Procédé selon l'une quelconque des revendications 1 à 6, caractérisé en ce que la réaction est effectuée à une température de 180°C à 260°C.

8. Procédé selon l'une quelconque des revendications 1 à 7, caractérisé en ce que l'hydrolyse du diester de propylène glycol en l'hydroxyester correspondant est effectuée par réaction du diester avec de l'eau ou avec le propylène glycol correspondant et que l'hydrolyse a lieu avant ou durant l'élimination de l'acide carboxylique de l'hydroxyester.

9. Procédé selon l'une quelconque des revendications 1 à 8, caractérisé en ce que l'élimination de l'acide carboxylique du monoester de propylène glycol est effectuée à une température de 160°C à 380°C, en présence d'une base, dans un solvant organique qui bout au-dessus de la température de réaction a la pression existante.

10. Procédé selon l'une quelconque des revendications 1 à 9, caractérisé en ce que l'acide carboxylique éliminé est recyclé à la première étape de réaction en tant que diluant pour la réaction pour la production d'oxyde de propylène à partir d'un 1,2-dihalogénopropane.